(19) Europäisches Patentamt
European Patent Office
Office européen des brevets

(11) Numéro de publication: **0 350 385**
**A1**

(12) # DEMANDE DE BREVET EUROPEEN

(21) Numéro de dépôt: **89401905.8**

(51) Int. Cl.$^5$: **A 61 K 7/13**

(22) Date de dépôt: **04.07.89**

(30) Priorité: **08.07.88 LU 87270**

(43) Date de publication de la demande:
**10.01.90 Bulletin 90/02**

(84) Etats contractants désignés:
**AT BE CH DE ES FR GB GR IT LI NL SE**

(71) Demandeur: **L'OREAL**
**14, Rue Royale**
**F-75008 Paris (FR)**

(72) Inventeur: **Bore, Pierre**
**197 Avenue Daniel Perdrigé**
**F-93370 Montfermeil (FR)**

**De Labbey, Arnaud**
**9, Rue Charles Dordain**
**F-93600 Aulnay-sous-Bois (FR)**

**Baudry, Alain**
**19, Villa du Buisson Ardent**
**F-95500 Gonesse (FR)**

(74) Mandataire: **Casalonga, Axel et al**
**BUREAU D.A. CASALONGA - JOSSE Morassistrasse 8**
**D-8000 München 5 (DE)**

(54) **Procédé de teinture des fibres kératiniques avec du 5,6-dihydroxyindole associé à un nitrite et composition de mise en oeuvre.**

(57) Procédé de teinture des fibres kératiniques avec du 5,6-dihydroxyindole associé à un nitrite et composition de mise en oeuvre.

La présente invention est relative à un nouveau procédé de teinture mettant en oeuvre le 5,6-dihydroxyindole associé à un nitrite, aux compositions mises en oeuvre dans ce procédé, à l'application de ce procédé à la teinture des cheveux humains et à des dispositifs à compartiments ou "kit" destinés à être utilisés pour la teinture.

EP 0 350 385 A1

**Description**

**Procédé de teinture des fibres kératiniques avec du 5,6-dihydroxyindole associé à un nitrite et composition de mise en oeuvre**

La présente invention est relative à un nouveau procédé de teinture mettant en oeuvre le 5,6-dihydroxyindole associé à un nitrite, aux compositions mises en oeuvre dans ce procédé.

Il est bien connu que la biosynthèse naturelle des eumélanines à partir de la tyrosine se fait en plusieurs étapes. L'une d'elles consiste en la formation du 5,6-dihydroxyindole qui s'oxyde pour former un pigment qui est l'un des constituants principaux de l'eumélanine.

On a déjà proposé dans le passé des procédés de teinture mettant en oeuvre le 5,6-dihydroxyindole et comportant deux étapes d'application. C'est ainsi que les brevets français de la demanderesse n° 1 133 594, 1 166 172, 2 390 158 et la demande de brevet français 2 536 993 proposent des procédés comportant l'application d'un cation métallique jouant le rôle de promoteur de la mélanogénèse dans une étape séparée de celle du 5,6-dihydroxyindole.

La demande de brevet français n° 2 594 331 de la demanderesse met en oeuvre, soit du manganèse sous forme de permanganate, soit un bichromate.

Dans le cas du cuivre et lorsqu'il est appliqué dans la première étape, l'imprégnation du cheveu se fait en milieu alcalin, alors qu'elle a lieu avec le permanganate en milieu acide ou neutre.

Généralement, le cheveu prétraité par le cation métallique est imprégné dans une deuxième étape par une solution alcaline ou neutre de 5,6-dihydroxyindole.

L'utilisation de cations métalliques dans les procédés de teinture pose cependant un certain nombre de problèmes dans la mesure où l'élimination de ces cations métalliques du cheveu est relativement difficile.

Après les deux traitements et malgré les rinçages, il reste toujours des traces de métaux sur les cheveux qui peuvent présenter des inconvénients lorsque les cheveux doivent subir des traitements ultérieurs comme des décolorations ou des permanentes. C'est plus particulièrement le cas du cuivre et du manganèse.

La demanderesse a, par ailleurs, également décrit, notamment dans sa demande de brevet français n° 2 593 061, un procédé de teinture au 5,6-dihydroxyindole mettant en oeuvre à la place d'un cation métallique un anion minéral et plus particulièrement un iodure.

Ce procédé est également mis en oeuvre en deux étapes. Au cours de la première étape, le cheveu est imprégné par une solution d'iodure de potassium et de 5,6-dihydroxyindole. Dans la seconde étape, les cheveux sont traités par application d'eau oxygénée. Cette dernière étape nécessaire qui met en oeuvre le peroxyde d'hydrogène, présente l'inconvénient bien connu d'être dégradant pour le cheveu et se rapproche des procédés classiques de teinture d'oxydation.

La demanderesse a découvert maintenant, de façon surprenante, qu'il était possible d'effectuer une teinture à l'aide du 5,6-dihydroxyindole, sans utiliser le peroxyde d'hydrogène, ni l'ammoniaque, ni un cation métallique, en imprégnant dans un premier temps, les fibres kératiniques et en particulier les cheveux par du 5,6-dihydroxyindole et dans un deuxième temps, par une composition acide, l'une au moins des compositions contenant un nitrite.

La demanderesse a découvert qu'il était facile de teindre les cheveux naturels dans des tons allant du gris en passant par le châtain jusqu'au noir intense.

L'invention a donc pour objet un nouveau procédé de teinture mettant en oeuvre du 5,6-dihydroxyindole et un nitrite.

Un autre objet de l'invention est constitué par des compositions ou des dispositifs à plusieurs compartiments utilisés dans le cadre de ce procédé.

D'autres objets de l'invention apparaîtront à la lecture de la description et des exemples qui suivent.

Le procédé de teinture des fibres kératiniques et en particulier des cheveux, conforme à l'invention, est essentiellement caractérisé par le fait que l'on applique sur ces fibres au moins une composition (A) contenant, dans un milieu approprié pour la teinture, au moins du 5,6-dihydroxyindole, l'application de la composition (A) étant suivie par l'application d'une composition aqueuse acide (B), la composition (A) ou la composition (B) contenant au moins un nitrite.

Les nitrites plus particulièrement utilisables conformément à l'invention sont :

- des nitrites de métaux alcalins, alcalino-terreux ou d'ammonium ou de tout autre cation cosmétiquement acceptable lorsqu'il est utilisé pour la teinture des cheveux humains vivants :
- dérivés organiques de nitrites tel que par exemple le nitrite d'amyle ;
- ou encore des vecteurs de nitrite c'est-à-dire des composés qui, par transformation, génèrent un nitrite.

Les nitrites particulièrement préférés sont les nitrites de sodium, de potassium ou d'ammonium.

Le 5,6-dihydroxyindole est utilisé dans la composition (A) dans des quantités suffisantes pour teindre, après association avec le nitrite, les fibres kératiniques suivant la nuance désirée. Cette concentration est comprise entre 0,01 et 0,3 mole/litre.

L'anion nitrite exprimé sous forme de $NO_2^-$ est présent dans des quantités suffisantes pour développer avec le 5,6-dihydroxyindole appliqué dans la première étape, une coloration. Sa concentration est de préférence comprise entre 0,02 et 1 mole/litre.

Le pH de la composition (A) est de préférence compris entre 2 et 10.

Le pH de la composition (B) est acide et permet de réguler la coloration.

En milieu acide, le nitrite est suffisamment oxydant pour réagir avec le 5,6-dihydroxyindole selon le schéma réactionnel suivant :

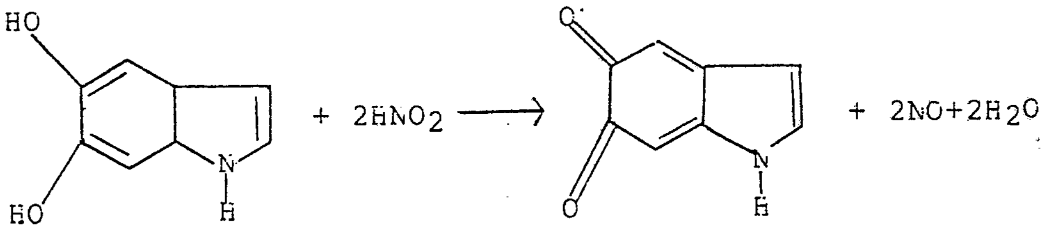


L'orthoquinone obtenue peut ensuite, par polymérisation, conduire à la formation du pigment.

En milieu alcalin, le nitrite n'est plus assez oxydant pour oxyder le 5,6-dihydroxyindole.

Le pH, conformément à l'invention, de la composition (B) est donc compris entre 2 et 6 et de préférence est ajusté à environ 3 pour des durées de contact faibles et pour les nuances les plus foncées.

Une forme de réalisation préférée de l'invention consiste à appliquer dans un premier temps la composition (A) contenant le 5,6-dihydroxyindole et dans un second temps la composition (B) contenant en milieu aqueux et acide, un nitrite.

Une autre forme de réalisation de l'invention peut consister à appliquer, dans un premier temps, une composition (A′) contenant dans un milieu approprié pour la teinture, du 5,6-dihydroxyindole en milieu neutre ou alcalin et un nitrite. Dans ce cas, on teint les cheveux en appliquant, dans un deuxième temps, une composition comprenant un milieu aqueux approprié pour la teinture, ajusté à un pH acide et de préférence compris entre 2 et 6.

Un autre objet de l'invention est donc constitué par une telle composition tinctoriale (A′) contenant simultanément le 5,6-dihydroxyindole et le nitrite. Les proportions en 5,6-dihydroxyindole et en nitrite ainsi que la nature des nitrites sont celles indiquées ci-dessus.

Les compositions (A) ou (B) ainsi que la composition (A′) contenant le 5,6-dihydroxyindole et le nitrite peuvent contenir des adjuvants habituellement utilisés en teinture des fibres kératiniques et plus particulièrement en plus de l'eau un solvant qui, dans le cas où la composition est appliquée sur les cheveux, est cosmétiquement acceptable.

Les solvants sont choisis parmi les solvants organiques, tels que les alcools inférieurs en $C_1$-$C_6$ comme l'alcool éthylique, l'alcool propylique ou isopropylique, l'alcool tertiobutylique, l'éthylèneglycol, le propylèneglycol, les éthers monométhylique, monoéthylique ou monobutylique de l'éthylèneglycol, l'acétate du monoéthyléther de l'éthylèneglycol, les monométhyléthers du propylèneglycol et du dipropylèneglycol, le lactate de méthyle.

Les solvants particulièrement préférés sont l'alcool éthylique et le propylèneglycol.

Les solvants sont utilisés plus particulièrement dans des concentrations comprises entre 10 et 20% pour les alcools in±érieurs lorsque la concentration du 5,6-dihydroxyindole dépasse 1% en poids par rapport au poids total de la composition.

Les compositions utilisables dans le procédé conforme à l'invention peuvent également contenir des agents tensio-actifs anioniques, cationiques, non ioniques, amphotères ou leurs mélanges, présents de préférence dans des proportions comprises entre 0,1 et 50%, des agents épaississants, des parfums, des agents séquestrants, des agents filmogènes, des agents de traitement, des agents dispersants, des agents de conditionnement, des agents conservateurs, des agents opacifiants, des agents de gonflement des fibres kératiniques.

Les épaississants peuvent être choisis plus particulièrement parmi l'alginate de sodium, la gomme arabique, la gomme de guar, les biopolymères comme la gomme de xanthane ou les scléroglucanes, les dérivés de cellulose tels que la méthylcellulose, l'hydroxyéthylcellulose, l'hydroxypropylméthylcellulose, le sel de sodium de la carboxyméthylcellulose et les polymères d'acide acrylique. On peut également utiliser des agents épaississants minéraux tels que la bentonite.

Ces épaississants, utilisés seuls ou en mélange, sont présents de préférence dans des proportions comprises entre 0,1 et 5% en poids par rapport au poids total de la composition et avantageusement entre 0,5 et 3% en poids.

Les agents d'acidification utilisables sont plus particulièrement choisis parmi l'acide lactique, l'acide acétique, l'acide tartrique, l'acide phosphorique, l'acide chlorhydrique, l'acide citrique.

Les agents alcalinisants sont plus particulièrement choisis parmi les amines, telles que les alcanolamines, les alkylamines ou alors les hydroxydes ou les carbonates alcalins ou d'ammonium.

Les compositions (A), (A′) et/ou (B) utilisables dans le procédé conforme à l'invention peuvent contenir d'autres colorants habituellement utilisés pour la teinture des fibres kératiniques et en particulier des colorants directs tels que les dérivés nitrés benzéniques, des colorants d'oxydation tels que des précurseurs de colorants d'oxydation de type para ou ortho, des coupleurs ou des colorants d'oxydation dits "rapides", c'est-à-dire des molécules à structure benzénique, précurseurs de colorants, susceptibles de générer les composés colorés par simple oxydation à l'air, pendant le temps de pose sur la chevelure, généralement

inférieur à 1 heure et en l'absence d'un autre agent oxydant.

Ces compositions peuvent être formulées sous des formes diverses, cosmétiquement acceptables, lorsqu'elles sont appliquées sur les cheveux, telles que de liquide plus ou moins épaissi ou gélifié, de crèmes, d'émulsions, de mousses ou d'autres formes appropriées pour réaliser la teinture.

En vue de la mise en oeuvre du procédé conforme à l'invention, on peut conditionner les différentes compositions dans un dispositif à plusieurs compartiments encore appelé "kit" ou nécessaire de teinture, comportant tous les composants destinés à être appliqués pour une même teinture sur les fibres kératiniques et en particulier les cheveux, en application successive avec ou sans prémélange.

De tels dispositifs sont connus en eux-mêmes et peuvent comporter un premier compartiment contenant la composition (A) contenant le 5,6-dihydroxyindole dans un milieu approprié pour la teinture, dans un deuxième compartiment, la composition (B) contenant dans un milieu approprié pour la teinture à un pH acide un nitrite tel que défini ci-dessus.

Une autre forme de réalisation consiste à prévoir un dispositif à plusieurs compartiments ou "kit" comportant dans un premier compartiment la composition (A') présentant un pH neutre ou alcalin, et comportant dans un milieu approprié pour la teinture du 5,6-dihydroxy-indole et un nitrite et dans un second compartiment, une solution aqueuse présentant un pH acide compris de préférence entre 2 et 6.

Le procédé conforme à l'invention ainsi que les compositions définies ci-dessus peuvent être mis en oeuvre pour teindre les cheveux naturels ou déjà teints, permanentés ou non ou défrisés, ou des cheveux fortement ou légèrement décolorés, éventuellement permanentés. Dans ce cas, on applique la composition (A) à une température qui est celle de la tête, c'est-à-dire comprise entre 25 et 35°C, pendant 5 à 30 minutes et on fait suivre, avec ou sans rinçage intermédiaire, cette application par l'application de la composition (B) contenant le nitrite qui est maintenue au contact des cheveux pendant 5 à 30 minutes. La température de teinture est également celle de la tête et elle est comprise entre 25 et 35°C.

Dans la forme de réalisation mettant en oeuvre le 5,6-dihydroxyindole et le nitrite dans une même composition en milieu neutre ou alcalin, on maintient la première composition (A') au contact des cheveux pendant 5 à 30 minutes et après un rinçage éventuel, on applique la composition aqueuse acide.

Il est également possible d'utiliser ces compositions pour la teinture des fourrures ou de la laine.

Les exemples suivants sont destinés à illustrer l'invention sans pour autant présenter un caractère limitatif.

## EXEMPLE 1

On imprègne une mèche de 1 g de cheveux naturels gris à 90% de blanc par une composition (A) suivante :

Composition (A)

| | |
|---|---|
| - 5,6-dihydroxyindole | 2,5 g |
| - Ethanol | 10,0 g |
| - CARBOPOL 940 (acide polyacrylique réticulé, vendu par la Société GOODRICH) | 2,0 g |
| - Eau qs | 100,0 g |

Le pH spontané est d'environ 5,5

La composition est maintenue au contact des cheveux pendant 15 minutes, suivi d'un rinçage à l'eau courante et d'un essorage.

On applique ensuite sur la mèche 5 ml de la composition (B) :

Composition (B)

| | |
|---|---|
| - Nitrite de sodium | 2,5 g |
| - Acide polyacrylamidométhylpropane sulfonique (solution à 20% de matière active) vendu par la société HENKEL sous la dénomination COSMEDIA POLYMER HSP 1180 | 25 g |
| - Acide chlorhydrique qs pH = 3 | |
| - Eau | qsp 100,0 g |

Cette composition est maintenue au contact des cheveux pendant 10 minutes.

On rince ensuite la mèche, on lave avec un shampooing à 5% de laurylsulfate de sodium. On obtient de cette façon une mèche dont la couleur est châtain noir.

## EXEMPLE 2

On imprègne une mèche de 1 g de cheveux par 5 ml d'une composition (A) suivante :

Composition (A)

| | |
|---|---|
| - 5,6-dihydroxyindole | 2,5 g |
| - Ethanol | 10,0 g |
| - Eau | qsp 100,0 g |

La composition est maintenue au contact des cheveux pendant 15 minutes.

On rince ensuite la mèche, on l'essore.

On imprègne ensuite la mèche par 5 ml d'une composition (B) :

Composition (B)

| | |
|---|---|
| - Nitrite de sodium | 0,2 g |
| - Acide citrique qs pH = 3 | |
| - Eau | qsp 100,0 g |

On maintient cette composition au contact des cheveux pendant 10 minutes, puis on rince.

On lave au shampooing avec une solution de laurylsulfate de sodium à 5%. On rince à nouveau et on sèche.

On obtient une coloration grise.

## EXEMPLE 3

On prépare la composition suivante :

| | |
|---|---|
| - 5,6-dihydroxyindole | 2,5 g |
| - Nitrite de sodium | 2,5 g |
| - Ethanol | 10,0 g |
| - Eau | qsp 100,0 g |

Le pH spontané est de 5,5

On applique, dans un premier temps, 5 ml de cette composition sur une mèche de 1g de cheveux décolorés.

On laisse en contact pendant 15 minutes à température ambiante, puis on rince à l'eau et on essore la mèche.

Dans un deuxième temps, on imprègne la mèche par 5 ml d'une solution aqueuse d'un tampon permettant d'ajuster le pH de la composition à 2 (acide citrique/HCl) pendant 10 minutes.

On rince, on lave au shampooing la mèche, on rince à nouveau et on sèche.

On obtient une coloration châtain moyen.

EXEMPLE 4

On applique sur une mèche de 1 g de cheveux gris à 90% de blancs, 10 g de la composition (A) définie dans l'exemple 1. On maintient cette composition au contact des cheveux pendant 15 minutes à température ambiante.

On rince et on essore la mèche.

On imprègne à nouveau cette mèche par 10 g d'une composition (B) suivante :

Composition (B)

| | |
|---|---|
| - Nitrite de sodium | 2,5 g |
| - Acide polyacrylamido méthyl propane sulfonique (à 20% de matière active) | 25 g |
| - Tampon (acide acétique/acétate de sodium) | qs pH 4,7 |
| - Eau | qsp 100,0 g |

On maintient cette composition (B) pendant 10 minutes au contact des cheveux.

On rince, on lave au shampooing, on rince à nouveau et on sèche.

On obtient une coloration châtain moyen avec de légers reflets violacés.

EXEMPLE 5

On prépare la composition suivante :

| | |
|---|---|
| - 5,6-dihydroxyindole | 2,0 g |
| - Ethanol | 10,0 g |
| - Hydroxyéthyl cellulose vendue par UNION CARBIDE sous la dénomination CELLOSIZE W.P 3 | 3,0 g |
| - Monoéthanolamine qs pH = 8,5 | |
| - Eau | qsp 100,0 g |

On applique 10 g de cette composition sur une mèche de 1 g de cheveux naturels à 90% de blancs. On laisse 15 minutes, on rince à l'eau courante et on essore.

On applique ensuite, pendant dix minutes, sur la mèche 10 g de la composition suivante :

| | |
|---|---|
| - Nitrite de sodium | 2,0 g |
| - Acide polyacrylamidométhyl propane sulfonique à 20% dans l'eau | 25,0 g |
| - HCl qs pH = 3 | |
| - Eau | qsp 100,0 g |

On rince, lave la mèche avec un shampooing, rince à nouveau, puis la sèche.

La mèche a une couleur châtain foncé.

EXEMPLE 6

On imprègne une mèche de 1 g de cheveux naturels à 90% de blancs par 10 g de la composition suivante :

| | |
|---|---|
| - 5,6-dihydroxyindole | 2,0 g |
| - Ethanol | 10,0 g |
| - CARBOPOL 940 | 2,0 g |
| - Tampon qs pH = 3 (acide citrique/HCl) | |
| - Eau | qsp 100,0 g |

On laisse au contact du cheveu pendant 15 minutes à température ambiante. On rince à l'eau courante, puis on essore.

On imprègne à nouveau la mèche par la composition contenant du nitrite et décrite à l'exemple 5.

On laisse dix minutes, on rince, lave au shampooing, rince à nouveau, puis sèche la mèche.

On obtient un châtain foncé.

## EXEMPLE 7

On applique sur une mèche de 1 g de cheveux gris à 90% de blancs, 10 g de la composition (A) définie dans l'exemple 1. On laisse dix minutes à température ambiante.

On élimine le gel avec un papier ou un linge. On imprègne à nouveau la mèche par 10 g d'une composition (B) définie dans l'exemple 1. On laisse dix minutes à température ambiante. On rince à l'eau, puis on lave au shampooing, on rince à nouveau et on sèche.

On obtient une coloration châtain noir.

## EXEMPLE 8

On applique sur une mèche de 1 g de cheveux décolorés 10 g de la composition (A) définie dans l'exemple 1. On maintient cette composition au contact du cheveu pendant quinze minutes à température ambiante. On rince et on essore la mèche.

On imprègne à nouveau cette mèche par 10 g de la composition suivante

| | |
|---|---|
| - Nitrite d'amyle | 4,5 g |
| - Ethanol | 20,0 g |
| - Acide polyacrylamidométhyl propane sulfonique à 20% dans l'eau | 25,0 g |
| - Eau | qsp 100,0 g |

On maintient cette composition pendant dix minutes au contact du cheveu. On rince, on lave au shampooing, on rince et sèche.

On obtient une coloration châtain/noir.

## EXEMPLE 9

On applique sur une mèche de 1 g de cheveux naturels à 90% de blancs permanentés 10 g de la composition suivante :

| | |
|---|---|
| - 5,6-dihydroxyindole | 2,0 g |
| - Ethanol | 10,0 g |
| - CARBOPOL 940 | 2,0 g |
| - Eau | qsp 100,0 g |

Le pH spontané est d'environ 5,5.

On laisse au contact du cheveu pendant 15 minutes à température ambiante. On lave et essore la mèche.

Sur cette même mèche, on applique alors 10 g de la composition (B) de l'exemple 1.

On laisse 10 minutes à température ambiante, on rince, on lave au shampooing, on rince à nouveau et on sèche la mèche.

On obtient une coloration châtain noir.

EXEMPLE 10

On applique sur une mèche de 1 g de cheveux décolorés, 10 g de la composition suivante :

| | |
|---|---|
| - 5,6-dihydroxyindole | 1,0 g |
| - Ethanol | 10,0 g |
| - CELLOSIZE W.P 3 (hydroxyéthyl cellulose) | 2,5 g |
| - Tampon pH = 10 | 10,0 g |
| - Eau | qsp 100,0 g |

On laisse 15 minutes, on rince et on essore la mèche.
On imprègne à nouveau la mèche par 10 g de la composition suivante :

| | |
|---|---|
| - Nitrite de sodium | 1,5 g |
| - Acide polyacrylamidométhyl propane sulfonique à 20% dans l'eau | 25,0 g |
| - Eau | qsp 100,0 g |

On laisse cette composition pendant 10 minutes au contact du cheveu. On rince, on lave au shampooing, on rince à nouveau et on sèche.
On obtient une coloration noire.

EXEMPLE 11

On applique sur une mèche de 1 g de cheveux naturels à 90% de blancs 10 g de la composition suivante :

| | |
|---|---|
| - 5,6-dihydroxyindole | 1,0 g |
| - Paraphénylène diamine | 1,0 g |
| - Ethanol | 20,0 g |
| - Carbopol 940 | 2,0 g |
| - Eau | qsp 100,0 g |

On laisse quinze minutes cette composition au contact du cheveu. On rince puis on essore la mèche.
On applique ensuite sur la mèche 10 g de la composition suivante :

| | |
|---|---|
| - Nitrite de sodium | 2,0 g |
| - Acide polyacrylamidométhyl propane sulfonique à 20% dans l'eau | 25,0 g |
| - HCl qs pH : 3 | |
| - Eau | qsp 100,0 g |

On laisse dix minutes, on rince, on lave au shampooing, on rince à nouveau et on sèche.
On obtient un châtain moyen.

EXEMPLE 12

On applique sur une mèche de 1 g de cheveux naturels à 90% de blancs 10 g de la composition suivante

| | |
|---|---|
| - 5,6-dihydroxyindole | 2,0 g |
| - Paraphénylène diamine | 1,0 g |
| - Resorcine | 1,0 g |
| - Ethanol | 20,0 g |
| - CARBOPOL 940 | 2,0 g |
| - Eau | qsp 100,0 g |

pH spontané d'environ 6.

On rince la mèche ensuite à l'eau courante, on l'essore et on l'imprègne à nouveau avec 10 g de la composition (B) décrite dans l'exemple 1.

On laisse en contact pendant 10 minutes, on rince à l'eau, puis on lave au shampooing, rince à nouveau et on sèche.

On obtient une mèche châtain foncé.

EXEMPLE 13

On imprègne pendant 15 minutes une mèche de 1 g de cheveux naturels à 90% de blancs par la composition suivante :

| | |
|---|---|
| - 5,6-dihydroxyindole | 1,0 g |
| - 1-N,N'-(β-dihydroxyéthyl)amino 3-nitro 4-(β-hydroxyéthyl)amino benzène | 0,5 g |
| - Ethanol | 20,0 g |
| - CELLOSIZE W.P 3 | 4,0 g |
| - Monoéthanolamine qs pH = 9 | |
| - Eau | qsp 100,0 g |

Au bout des 15 minutes de contact, on lave, puis on essore la mèche. On imprègne à nouveau la mèche pendant 10 minutes avec la composition suivante :

| | |
|---|---|
| - Nitrite de sodium | 2,0 g |
| - Acide polyacrylamidométhyl propane sulfonique (solution à 20% dans l'eau) | 25,0 g |
| - HCl qs pH = 3 | |
| - Eau | qsp 100,0 g |

Après contact, on lave la mèche à l'eau courante, on effectue un shampooing au laurylsulfate de sodium à 5%, on rince à nouveau et on sèche.

On obtient une mèche dont la coloration est châtain doré.

EXEMPLE 14

Appliquer pendant quinze minutes sur une mèche de 1 g de cheveux naturels à 90% de blancs la composition suivante :

| | |
|---|---|
| - 5,6-dihydroxyindole | 4,5 g |
| - Ethanol | 20,0 g |
| - Carbopol 940 | 2,0 g |
| - Eau | qsp 100,0 g |

Après les quinze minutes de temps de pose, rincer la mèche à l'eau courante puis l'essorer. imprégner à nouveau cette mèche pendant dix minutes par la composition suivante :

| | |
|---|---|
| - Nitrite de sodium | 0,15 g |
| - Acide polyacrylamidométhyl propane sulfonique à 20% dans l'eau | 25,0 g |
| - HCl qs pH : 3 | |
| - Eau | qsp 100,0 g |

Après les dix minutes de contact, rincer la mèche à l'eau courante, effectuer un shampooing au laurylsulfate de sodium à 5%, rincer à nouveau et sécher.

On obtient une mèche dont la couleur est gris foncé.

**Revendications**

1. Procédé de teinture des fibres kératiniques, caractérisé par le fait que l'on applique sur ces fibres au moins une composition (A) contenant dans un milieu approprié pour la teinture au moins du 5,6-dihydroxyindole, l'application de la composition (A) étant suivie par l'application d'une composition (B) constituée par une composition aqueuse présentant un pH acide, la composition (A) ou (B) contenant au moins un nitrite.

2. Procédé de teinture selon la revendication 1, caractérisé par le fait que le nitrite est choisi parmi les nitrites de métaux alcalins, alcalino-terreux ou d'ammonium ou d'un autre cation cosmétiquement acceptable, ainsi que des dérivés organiques de nitrites ou des vecteurs de nitrites générant par transformation un nitrite.

3. Procédé selon la revendication 1 ou 2, caractérisé par le fait que le nitrite est choisi parmi les nitrites de sodium, de potassium ou d'ammonium.

4. Procédé selon l'une quelconque des revendications 1 à 3, caractérisé par le fait que le 5,6-dihydroxyindole est utilisé dans des concentrations comprises entre 0,01 et 0,3 mole/litre.

5. Procédé selon l'une quelconque des revendications 1 à 3, caractérisé par le fait que le nitrite est utilisé dans des proportions comprises entre 0,02 et 1 mole/litre.

6. Procédé selon l'une quelconque des revendications 1 à 5, caractérisé par le fait que le pH de la composition (A) est compris entre 2 et 10.

7. Procédé selon l'une quelconque des revendications 1 à 5, caractérisé par le fait que le pH de la composition (B) est compris entre 2 et 6.

8. Procédé selon l'une quelconque des revendications 1 à 7, caractérisé par le fait que la composition (A) ou (B) contient en plus de l'eau au moins un ou plusieurs solvant(s) organique(s) cosmétiquement acceptable(s).

9. Procédé de teinture des fibres kératiniques selon l'une quelconque des revendications 1 à 8, caractérisé par le fait que l'on applique dans un premier temps une composition (A) contenant, dans un milieu approprié pour la teinture, le 5,6-dihydroxyindole et dans un second temps une composition (B) contenant dans un milieu aqueux ajusté à un pH acide, un nitrite.

10. Procédé selon l'une quelconque des revendications 1 à 8, caractérisé par le fait que l'on applique dans un premier temps, une composition (A′) contenant dans un milieu approprié pour la teinture et présentant un pH neutre ou alcalin, du 5,6-dihydroxyindole et un nitrite, et dans un deuxième temps, on applique une composition aqueuse ajustée à un pH acide.

11. Procédé selon l'une quelconque des revendications 1 à 10, caractérisé par le fait que la composition A, A′ ou B contient en plus un colorant direct, un colorant d'oxydation, un coupleur ou un colorant d'oxydation rapide.

12. Procédé selon l'une quelconque des revendications 1 à 11, caractérisé par le fait que l'on fait suivre la première étape par une étape de rinçage.

13. Procédé selon l'une quelconque des revendications 1 à 12, caractérisé par le fait que la composition appliquée dans le premier temps est maintenue au contact des cheveux pendant une durée de 5 à 30 minutes et que la composition appliquée dans le second temps est maintenue au contact des cheveux pendant une durée également de 5 à 30 minutes.

14. Application du procédé tel que défini dans l'une quelconque des revendications 1 à 13, à la teinture des cheveux humains.

15. Composition destinée à être utilisée pour la teinture des fibres kératiniques et en particulier des cheveux humains, caractérisée par le fait qu'elle contient dans un milieu aqueux à pH neutre ou alcalin, approprié pour la teinture, au moins du 5,6-dihydroxyindole et au moins un nitrite.

16. Dispositif à plusieurs compartiments ou "kit" destiné à être utilisé dans la teinture des fibres kératiniques et en particulier des cheveux humains, caractérisé par le fait qu'il comporte dans un premier

compartiment une composition (A) contenant dans un milieu approprié pour la teinture du 5,6-dihydroxyindole et dans un compartiment (B) une composition aqueuse présentant un pH acide contenant un nitrite.

17. Dispositif à plusieurs compartiments ou "kit" destiné à être utilisé dans la teinture des fibres kératiniques et en particulier les cheveux humains, caractérisé par le fait qu'il comporte dans un premier compartiment une composition (A') présentant un pH neutre ou alcalin et comportant, dans un milieu approprié pour la teinture, du 5,6-dihydroxy-indole et un nitrite, et dans un second compartiment une composition aqueuse acide.

Office européen des brevets

# RAPPORT DE RECHERCHE EUROPEENNE

Numero de la demande

EP 89 40 1905

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | GB-A-2 185 498 (L'OREAL)<br>* Revendications * | 1-17 | A 61 K 7/13 |
| A | HOUBEN-WEYL: "Methoden der organischen Chemie", vol. VII/3b, Chinone, partie II, 1979, pages 32-35, Georg Thieme Verlag, Stuttgart, DE<br>* En entier * | 1-17 | |
| A | L.F. FIESER et al.: "Reagents for organic synthesis", 1967, pages 1097-1101, John Wiley and Sons, Inc., New York, US<br>* En entier * | 1-17 | |
| A | EP-A-0 148 466 (KAO CORP.)<br>* Page 4, ligne 7 - page 5, ligne 2; revendications * | 1-17 | |
| A | CHEMICAL ABSTRACTS, vol. 89, no. 1, 3 juillet 1978, page 200, résumé no. 2111y, Columbus, Ohio, US; F.N. BOCTOR et al.: "A colorimetric method for the determination of indole, and its application to assay of tryptophanas e", & ANAL. BIOCHEM. 1978, 86(2), 457-62<br>* Résumé * | 1-17 | **DOMAINES TECHNIQUES RECHERCHES (Int. Cl.5)**<br>A 61 K |

Le présent rapport a été établi pour toutes les revendications

| Lieu de la recherche | Date d'achèvement de la recherche | Examinateur |
|---|---|---|
| LA HAYE | 19-09-1989 | GOETZ G. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)